# EUROPEAN PATENT APPLICATION

(11) **EP 4 621 046 A1**
(43) Date of publication of application: **24.09.2025**
(21) Application number: 23891683.7
(22) Date of filing: 17.11.2023
(51) Int. Cl.: C12N 5/0783, A61K 35/17, A61P 35/00, A61P 37/04, C12N 15/09

(54) **PRODUCTION AND CRYOPRESERVATION METHOD FOR LYMPHOCYTES, CLEANSING LIQUID AND CRYOPRESERVATION LIQUID FOR SAME, AND PREPARATION OF LYMPHOCYTES FOR TRANSPLANTATION FROM CRYOPRESERVED LYMPHOCYTES**

(30) Priority: 18.11.2022 JP 2022185201
(71) Applicant: Healios K.K., Tokyo 100-0006 (JP)
(72) Inventor: HINO, Motohiro, Tokyo 100-0006 (JP); FUKUDA, Takayuki, Tokyo 100-0006 (JP); CHANG, Yun Hsiang, Tokyo 100-0006 (JP)
(74) Representative: Weickmann & Weickmann PartmbB
(86) International application number: PCT/JP2023/041479
(87) International publication number: WO 2024/106538

(57) **Abstract**

The present invention provides a method for washing lymphocytes, including washing lymphocytes with a bicarbonate ion-containing physiological aqueous solution; a method for cryopreserving lymphocytes, including suspending and freezing lymphocytes obtained by the method in a cryopreservation solution containing a physiological aqueous solution; a washing solution for lymphocytes, including a bicarbonate ion-containing physiological aqueous solution; a cryopreservation solution for lymphocytes, containing a bicarbonate ion-containing physiological aqueous solution, dextran, glucose, DMSO, and human serum albumin; a lymphocyte-containing composition, containing lymphocytes treated with a bicarbonate ion-containing physiological aqueous solution and the aforementioned cryopreservation solution; a kit for preparing lymphocytes for transplantation, containing the lymphocyte-containing composition and a diluent for transplantation containing a bicarbonate ion-containing physiological aqueous solution, and the like.

## Description

### [Technical Field]

The present invention relates to a method for washing and cryopreserving lymphocytes such as T cells and natural killer cells (hereinafter sometimes to be abbreviated as "NK cells"), which are useful in the medical field, a washing solution and a cryopreservation solution therefor, a lymphocyte-containing composition to be washed and cryopreserved using the method or solution, a diluent for transplantation for the lymphocyte-containing composition, a kit for preparing lymphocytes for transplantation, which contains the lymphocyte-containing composition and the diluent for transplantation, a method for preparing lymphocytes for transplantation, which uses the kit, and a lymphocyte preparation for transplantation, which is obtained by the method. The washing solution and the diluent for transplantation contain a bicarbonate ion-containing physiological aqueous solution, and the cryopreservation solution contains a physiological aqueous solution, preferably a bicarbonate ion-containing physiological aqueous solution.

### [Background Art]

Cells involved in cancer immunity include lymphocytes such as T cell, NK cell, natural killer T (NKT) cell and the like. In recent years, developments of induced pluripotent stem cells (iPS cells) and methods for inducing differentiation therefrom, as well as technology developments such as gene modification methods of pluripotent stem cells such as iPS cell and the like have made it possible to genetically modify an iPS cell and introduce differentiation into an immune cell of interest, thereby producing an immune cell on which high function is conferred. As cell pharmaceuticals containing genetically modified immune cells, those using CAR-T cell targeting CD19 (Kymriah and Yescarta) have been approved to date. On the other hand, since NK cells have unique properties different from T cells, expectation for immune cell therapy using NK cells is growing, and various clinical tests targeting cell medicines using modified NK cells are actually being conducted.

In order to use immune cells as cell medicines, it is necessary to prepare a large amount of immune cells. There are many reports on methods for inducing differentiation of lymphocytes such as T cells and NK cells from pluripotent stem cells and methods for expansion culturing lymphocytes. For example, the applicant developed a technology for efficiently producing large amounts of NK cells that maintain high activity even after freezing and thawing, which technology including forming pluripotent stem cell spheres of a more preferred size by three-dimensional suspension culture, inducing differentiation of hematopoietic progenitor cells from the spheres by performing medium exchange by perfusion (continuous three-dimensional perfusion culture), and differentiating the obtained hematopoietic progenitor cells into NK cells by three-dimensional suspension culture, and filed a patent application for the technology (Patent Literature 10).

Since the culture of cells for transplantation must be performed at a GMP level, when immune cells are used as cell medicines, it is a general practice to transport cells produced and frozen at a cell processing center (CPC) to a hospital, thaw the cells in the hospital, suspend and dilute them in a medium for transplantation, and then bring them to an operating room and transplant the cells. As a cryopreservation solution, a medium for cell culture, to which a cryoprotectant has been added, has been used. However, several dozen types of the medium components therein may include those harmful to the recipient, which makes it necessary to wash the cells after thawing and prior to transplantation, causing problems such as time loss and reduced cell recovery rate and survival rate.

In response to those problems, efforts have been made to develop washing solutions and cryopreservation solutions that can wash cells before freezing to remove harmful components and maintain high cell recovery rates and survival rates even after freezing and thawing. There have been reports on the use of solutions based on physiological aqueous solutions, such as saline and Ringer's solution, as washing solutions and cryopreservation solutions. For example, it has been reported that, in the washing and cryopreservation steps of human mesenchymal stem cells, a decrease in the cell survival rate in "each step" can be suppressed by the use of a solution containing human serum albumin (HuSA) and bicarbonate Ringer's solution (Patent Literature 1). In addition, it has been reported that a solution containing glucose and/or HuSA and bicarbonate is used as a preservation solution for long-term storage of cells in a non-frozen state (refrigerated or at room temperature), in order to avoid the use of highly concentrated and harmful cryoprotectants such as dimethyl sulfoxide (DMSO) and to apply the solution to cells such as NK cells that have low storage stability after cryopreservation (Patent Literatures 2 and 3). However, there have been no reports on the use of a bicarbonate solution as a washing solution before cryopreservation of lymphocytes, and there have been no reports of investigating the influence of the washing solution on the recovery rate and survival rate of cells after freezing and thawing, not after washing.

As the cryopreservation solution for lymphocyte, the use of solutions containing some of DMSO, dextran, glucose, HuSA, bicarbonate, and physiological aqueous solution (e.g., saline, Ringer's solution) has been reported (Patent Literatures 1, 5-9). However, a cryopreservation solution containing all of the aforementioned components or consisting of the aforementioned components has not been disclosed.

### [Citation List]

### [Patent Literature]

[Patent Literature 1]
   WO 2009/057537
[Patent Literature 2]
   JP 2008-104407 A
[Patent Literature 3]
   WO 2013/115322
[Patent Literature 4]
   JP 2018-138565 A
[Patent Literature 5]
   WO 2011/021618
[Patent Literature 6]
   JP 2000-201672 A
[Patent Literature 7]
   JP 2018-183161 A
[Patent Literature 8]
   WO 2018/084228
[Patent Literature 9]
   CN 112868642 A
[Patent Literature 10]
   WO 2023/145922

### [Summary of Invention]

### [Technical Problem]

As described above, in order to produce lymphocytes for transplantation, washing solutions and cryopreservation solutions capable of maintaining high cell recovery rates and survival rates even after freezing and thawing have been developed intensively. However, they have not necessarily been satisfactory. In particular, it has not been assumed to take note of the washing step of lymphocytes for transplantation and maintain or improve high cell recovery rates and survival rates after freezing and thawing. Therefore, an object of the present invention is to provide a washing solution and a cryopreservation solution for lymphocytes that maintain high cell recovery rates and survival rates even after freezing and thawing, and can produce lymphocytes for transplantation that maintain high physiological activity. Another object of the present invention is to provide a diluent for transplantation that can maintain high cell survival rates even after thawing a frozen lymphocyte-containing composition obtained using the washing solution and cryopreservation solution and storing the frozen lymphocyte-containing composition at room temperature until transplantation, and to provide a lymphocyte preparation that is more suitable for transplantation by using a kit for preparing lymphocytes for transplantation that includes the lymphocyte-containing composition and the diluent for transplantation.

### [Solution to Problem]

The present inventors have conducted intensive studies in an attempt to achieve the above-mentioned purposes and surprisingly found that, when lymphocytes after maintenance and expansion culture are suspended and left standing (simulating a washing step) in a physiological aqueous solution containing bicarbonate ion such as bicarbonate Ringer's solution, and then frozen and stored in a conventionally-known cryopreservation solution, the cell recovery rate and cell survival rate after thawing and recovery culture compared to those before cryopreservation remarkably increase compared to when the cells are suspended and left standing in a physiological aqueous solution not containing bicarbonate ion.

The present inventors then suspended and cryopreserved lymphocytes after maintenance and expansion culture in cryopreservation solutions containing dextran, glucose, DMSO, HuSA, and a physiological aqueous solution (e.g., saline, bicarbonate Ringer's solution, etc.) at various concentrations to find that the cryopreservation solutions containing each of the aforementioned components in a given concentration range remarkably improved the viable cell recovery rate after thawing compared to generally used cryopreservation solutions. In addition, when cells were suspended in the cryopreservation solution containing bicarbonate Ringer's solution as a physiological aqueous solution and left standing at room temperature for 0-4 hr before cryopreservation, a decrease in the viable cell recovery rate after thawing and recovery culture over time by standing still at room temperature was remarkably suppressed.

Furthermore, in order to investigate the effect of physiological aqueous solutions as a transplantation medium (diluent), the present inventors thawed frozen NK cells, diluted the cell suspension with various physiological aqueous solutions, and left standing at room temperature for 0-4 hr. As a result, they have found that the viable cell recovery rate was remarkably improved when a bicarbonate ion-containing physiological aqueous solution was used, compared to a physiological aqueous solution not containing said ion.

Based on these findings, the present inventors have conducted further studies and completed the present invention.

Accordingly, the present invention provides the following.

### [Item 1]

A method for washing lymphocytes, comprising washing lymphocytes with a bicarbonate ion-containing physiological aqueous solution.

### [Item 2]

The method of Item 1, wherein the lymphocyte is a T cell or NK cell.

### [Item 3]

The method of Item 1 or 2, wherein the bicarbonate ion-containing physiological aqueous solution is bicarbonate Ringer's solution.

### [Item 4]

A method for cryopreserving lymphocytes, comprising suspending and freezing lymphocytes treated by the method of any one of Items 1 to 3 in a cryopreservation solution containing a physiological aqueous solution.

### [Item 5]

The method of Item 4, wherein the physiological aqueous solution is a bicarbonate ion-containing physiological aqueous solution.

### [Item 6]

The method of Item 5, wherein the bicarbonate ion-containing physiological aqueous solution is bicarbonate Ringer's solution.

### [Item 7]

A washing solution for lymphocytes, comprising a bicarbonate ion-containing physiological aqueous solution.

### [Item 8]

A cryopreservation solution for lymphocytes, comprising a bicarbonate ion-containing physiological aqueous solution, dextran, glucose, DMSO, and human serum albumin.

### [Item 9]

A lymphocyte-containing composition, comprising lymphocytes treated with a bicarbonate ion-containing physiological aqueous solution and the cryopreservation solution of Item 8.

### [Item 10]

A kit for preparing lymphocytes for transplantation, comprising the lymphocyte-containing composition of Item 9 and a diluent for transplantation containing a bicarbonate ion-containing physiological aqueous solution.

### [Item 11]

A diluent for transplantation for the lymphocyte-containing composition of Item 9, the diluent comprising a bicarbonate ion-containing physiological aqueous solution.

### [Item 12]

A method for preparing lymphocytes for transplantation, comprising diluting the lymphocyte-containing composition of Item 9, which has been cryopreserved and then thawed, with a diluent for transplantation containing a bicarbonate ion-containing physiological aqueous solution.

### [Item 13]

A lymphocyte preparation for transplantation, which is obtained by the method of Item 12.

### [Advantageous Effects of Invention]

According to the washing solution and the cryopreservation solution of the present invention, high cell recovery rates and survival rates can be maintained even after freezing and thawing, and lymphocytes (e.g., NK cell, T cell, etc.) for transplantation that maintain high physiological activity can be produced. Furthermore, the lymphocyte-containing composition and the diluent for transplantation of the present invention can maintain a high cell survival rate even when stored at room temperature after thawing until transplantation, and can provide a lymphocyte preparation more suitable for transplantation.

### [Description of Embodiments]

### [I] The washing method of the present invention and the washing solution of the present invention

The present invention provides a method for washing lymphocytes, including washing lymphocytes with a bicarbonate ion-containing physiological aqueous solution (hereinafter sometimes referred to as the "washing method of the present invention").

In the present specification, "washing" refers to the operation of suspending cells in a liquid (i.e., washing solution) in order to remove substances (e.g., medium components) present in the surrounding environment (e.g., culture medium) of the cells before washing. Washing of cells can be performed, for example, by removing the dispersion medium of the cells before washing (e.g., centrifugation, filtration, etc.) and suspending the collected cells in the washing solution (the operation is repeated as necessary), or by replacing the dispersion medium before washing with the washing solution using, for example, an automatic concentration and washing device, and the manner of washing is not particularly limited.

### (A) Lymphocyte

The lymphocytes to be subjected to the washing method of the present invention are not particularly limited, and examples thereof include NK cells, T cells (e.g., cytotoxic T cells (CTL), helper T cells, regulatory T cells, etc.), and B cells. They are preferably NK cells and T cells, more preferably NK cells. Lymphocytes can be isolated from blood (e.g., peripheral blood, umbilical cord blood, etc.) collected from humans or other mammals (preferably humans) or mononuclear cells separated therefrom, by using a conventional method such as flow cytometry, and using the expression of surface antigen markers (e.g., CD3, CD4, CD8, etc. in the case of T cells, CD56, etc. in the case of NK cells) that are specifically expressed in the lymphocytes of interest as an index.

In one preferred embodiment, various lymphocytes can be induced to differentiate from lymphocyte progenitor cells, including pluripotent cells. Examples of such lymphocyte progenitor cells include iPS cells, embryonic stem cells (ES cells), embryonic carcinoma cells (EC cells), embryonic germ cells (EG cells), hematopoietic stem cells, multipotent progenitors (MPPs) that have lost the ability to self-replicate, myelo-lymphoid common progenitors (MLPs), myeloid progenitors (MPs), granulocyte mononuclear progenitors (GMPs), macrophage-dendritic cell precursors (MDPs), dendritic cell precursors (DCPs), and the like. Preferred are pluripotent stem cells such as ES cells and iPS cells.

As a method for inducing differentiation of the aforementioned progenitor cells into lymphocytes, various known differentiation induction methods can be appropriately selected and used. For example, a known method for inducing differentiation from human pluripotent stem cells into T cells is, for example, the method described in Timmermans, F. et al., J. Immunol., 2009, 182, 68879-6888. In addition, human iPS cells established from human peripheral blood T lymphocytes can be induced to differentiate into T cells by, for example, the method described in Nishimura, T. et al. Cell Stem Cell, 2013, 12, 114-126. Furthermore, known methods for inducing differentiation from human pluripotent stem cells into NK cells include, for example, the methods described in WO 2020/086889, Biochem Biophys Rdeterminees Commun. 515(1): 1-8 (2019), and Methods Mol Biol. 2048: 107-119 (2019).

T cells can be activated and expanded by a conventional method from T cell-containing samples such as PBMCs or T cells induced to differentiate from lymphocyte progenitor cells. For example, a molecule that interacts with a surface molecule of T cells to promote activation and/or proliferation of T cells can be added to the medium of a T cell-containing sample and cultured. Examples of such molecules include CD3, which conjugates with TCR and is responsible for TCR-mediated signal transduction, and molecules that specifically bind to surface molecules CD28, ICOS, CD137, OX40, CD27, GITR, BAFFR, TACI, BMCA, CD40L, and the like, known as costimulatory factors for T cell activation, and have the function of transmitting activation/proliferation signals or co-signals into T cells. Preferred examples include antibodies against CD3 and/or antibodies against CD28. These molecules may be used alone or in the form of a complex (e.g., TransAct (Milteny Biotech), Dynabeads Human T-Activator CD3/CD28 (ThermoFisher Scientific)) bound to a carrier (e.g., Dynabeads (registered trademark) (ThermoFisher Scientific)). When stimulating the proliferation of T cells by using anti-CD3 antibody and/or anti-CD28 antibody, cytokines such as IL-2, IL-7, and IL-15 may be further added to the medium.

NK cells can be activated and expanded by a conventional method from T cell-containing samples such as PBMCs or NK cells induced to differentiate from lymphocyte progenitor cells. Examples of such methods include, but are not limited to, the methods described in WO 2020/086889, Biochem Biophys Res Commun. 515(1): 1-8 (2019), and Methods Mol Biol. 2048: 107-119 (2019).

As described above, the present applicant developed a method for efficiently producing large amounts of NK cells that maintain high activity even after freezing and thawing, by inducing differentiation of hematopoietic progenitor cells from the pluripotent stem cell spheres by continuous three-dimensional perfusion culture, and differentiating the obtained hematopoietic progenitor cells into NK cells by three-dimensional suspension culture (JP Patent Application No. 2022-013646). More specifically, the method characteristically includes
(1) a step of forming pluripotent stem cell spheres having an average particle size of not less than 200 µm in a first medium;
(2) a step of inducing the pluripotent stem cell spheres formed in step (1) into a cell population including hematopoietic progenitor cells by three-dimensional culture using a second medium; and
(3) a step of inducing the cell population including hematopoietic progenitor cells obtained in step (2) into a cell population including NK cells by three-dimensional culture using a third medium,
wherein the steps (1) to (3) are performed by a three-dimensional perfusion culture method.

The three-dimensional floating culture method is one of the cell culture methods and characterized by culturing floating cells, spheres (spheroids), or carriers for three-dimensional culture with cells attached thereto, while developing them three-dimensionally using a stirring blade or shaker. In addition, the perfusion culture method is one of the continuous culture methods, and can achieve continuous supply of new nutrients and removal of waste products, which are the purposes of medium exchange, by simultaneously supplying a given amount of new medium to the culture medium in the culture container and removing a given amount of the medium. In this method, perfusion culture is preferably performed continuously. The "continuous perfusion culture" here refers to the process of simply replacing the membrane between each step, without a washing solution step generally performed when exchanging the medium, and supplying the medium for the next step while removing the medium from the previous step.

The separation membrane used during perfusion culture desirably have a certain fine pore size and characteristics (e.g., hydrophobicity) that do not allow the cell population of interest to escape from the culture system. Appropriate fine pore sizes include a fine pore size of 15-75 µm for step (1), a fine pore size of 45-225 µm for step (2), and a fine pore size of 0.2-10 µm for step (3).

The first medium in step (1) is not particularly limited as long as it is a culture medium used for the maintenance culture of pluripotent stem cells, and, for example, a medium capable of feeder-free culture is used. Preferably, it is a medium capable of feeder-free culture and containing a ROCK inhibitor.

The second medium in step (2) is not particularly limited as long as it is a medium capable of inducing pluripotent stem cells into hematopoietic progenitor cells, and examples thereof include a medium containing vascular endothelial growth factor (VEGF), bone morphogenetic protein 4 (BMP4), and a glycogen synthase 3β (GSK3β) inhibitor, a medium containing stem cell factor (SCF) and a transforming growth factor β (TGFβ)/Smad inhibitor, and a medium containing SCF and Flt3 ligand (Flt3L).

The third medium in step (3) is not particularly limited as long as it is a medium capable of inducing hematopoietic progenitor cells into NK cells, and examples thereof include a medium containing IL-15 and SCF.

The average sphere particle size in step (1) is appropriately adjusted within the range of generally 200 µm or more, specifically 200 to 600 µm. The culture period required for sphere formation is preferably about 2 to 10 days, more preferably about 4 to 7 days, from cell seeding.

The culture period in step (2) is generally 5 to 20 days, preferably about 10 to 18 days.

The culture period in step (3) is generally 20 to 60 days, preferably about 25 to 40 days.

A step of expansion culture of the obtained NK cells or a step of maturation of NK cells can be further performed as step (4) after step (3). The expansion culture step or the maturation step can be performed by appropriately applying a known method.

In the above-mentioned method, T cells can also be induced from pluripotent stem cells by performing, instead of step (3), a method known per se for inducing differentiation of hematopoietic stem cells into T cells.

### (B) Bicarbonate ion-containing physiological aqueous solution

In the present specification, "physiological aqueous solution" refers to an isotonic solution in which the salt and sugar concentrations are adjusted with sodium, potassium, etc. such that the osmotic pressure is approximately the same as that of body fluids (e.g., plasma) or cell fluids. Examples of isotonic solution include saline, phosphate buffered saline (PBS), Tris buffered saline (TBS), HEPES buffered saline, Ringer's solution, Hank's Balanced Salt Solution (HBSS), and 5% glucose aqueous solution. In the present specification, "isotonic" means that the osmotic pressure is within the range of 250 to 380 mOsm/L.

The bicarbonate ion-containing physiological aqueous solution used in the washing method of the present invention (hereinafter sometimes referred to as the "washing solution of the present invention") can be any physiological aqueous solution containing salts of bicarbonate ions and various cations. Examples of such bicarbonate salts include sodium bicarbonate, potassium bicarbonate, ammonium bicarbonate, and calcium bicarbonate. Preferred examples of the bicarbonate ion-containing physiological aqueous solution include bicarbonate Ringer's solution and HBSS. More preferred is bicarbonate Ringer's solution.

The "bicarbonate Ringer's solution" in the present specification is a physiological aqueous solution that contains bicarbonate ion, sodium ion, potassium ion, calcium ion, and chloride ion as electrolytes, and has a bicarbonate ion concentration of 22 to 31 mEq/L and a sodium ion concentration of 120 to 150 mEq/L. The pH of the bicarbonate Ringer's solution can be 6.8 to 7.8. The osmotic pressure ratio to saline may be, but is not limited to, 0.9 to 1.1. The bicarbonate Ringer's solution may further contain magnesium ions and/or citrate ions as electrolytes. When the bicarbonate Ringer's solution contains magnesium ions and citrate ions, the concentration of each electrolyte may be, for example, but are not limited to, as follows:
sodium ion 120 - 150 mEq/L
potassium ion 3.6 - 4.4 mEq/L
calcium ion 2.7 - 3.3 mEq/L
magnesium ion 0.9 - 2.2 mEq/L
chlorine ion 100 - 125 mEq/L
bicarbonate ion 22 - 31 mEq/L
citrate ion 3.6 - 5.5 mEq/L

Specific examples of such bicarbonate Ringer's solutions include the commercially available BICANATE (registered trademark; Otsuka Pharmaceutical Factory, Inc.) and BICARBON (registered trademark; AY PHARMACEUTICALS CO., LTD.). The concentration of each electrolyte therein is as follows. The composition (w/v%) of each component (salt) of these bicarbonate Ringer's solutions is as shown in Table 8.

### (BICANATE (registered trademark))

sodium ion 130 mEq/L
potassium ion 4 mEq/L
calcium ion 3 mEq/L
magnesium ion 2 mEq/L
chlorine ion 109 mEq/L
bicarbonate ion 28 mEq/L
citrate ion 4 mEq/L

### (BICARBON (registered trademark))

sodium ion 135 mEq/L
potassium ion 4 mEq/L
calcium ion 3 mEq/L
magnesium ion 1 mEq/L
chlorine ion 113 mEq/L
bicarbonate ion 25 mEq/L
citrate ion 5 mEq/L

Examples of HBSS include HBSS(-) which contains bicarbonate ion, sodium ion, potassium ion, chloride ion, and phosphate ion as electrolytes and has the electrolyte composition shown in Table 1, and HBSS(+) which further contains calcium ion and magnesium ion and has the electrolyte composition shown in Table 1. HBSS(+) is more preferred.

The washing solution of the present invention may contain components other than the above-mentioned electrolytes. Examples of such components include electrolytes (buffering agents) other than those mentioned above, such as phosphate ion, lactate ion, and acetate ion; sugars such as glucose, galactose, fructose, mannose, maltose, and sucrose; and proteins derived from living organisms, such as serum albumin (e.g., HuSA, BSA, etc.) and serum globulin.

However, in a preferred embodiment, the washing solution of the present invention is a bicarbonate ion-containing physiological aqueous solution not containing buffers, such as phosphate ion, lactate ion, and acetate ion, sugars (e.g., glucose, etc.), or biologically derived proteins (e.g., HuSA, etc.).

### (C) Washing method

In the washing method of the present invention, the method of washing lymphocytes with the washing solution of the present invention is not particularly limited, and can be performed, for example, by removing the dispersion medium (e.g., culture medium) of the lymphocytes before washing by centrifugation, filtration, and the like, and suspending the collected lymphocytes in the washing solution of the present invention (this operation is repeated as necessary).

In one preferred embodiment, for example, an automatic concentration and washing device that includes a cell filter composed of a hollow fiber membrane module or the like in the circuit and can feed a cell suspension and a washing solution into the circuit is used, and a series of operations including priming (filling the circuit with washing solution), concentration (feeding a cell suspension into the circuit and circulating it through the hollow fiber membrane while removing medium components (e.g., low molecular weight, protein, etc.) outside the membrane), washing (feeding a washing solution into the circuit and replacing same with medium components), and recovery (recovering the cell concentrate from which the medium components have been removed) are performed in a closed system, thereby making it possible to wash the cells in a dispersed state (removal of medium components) while reducing the risk of cell damage and contamination. Such automatic concentration and washing devices are commercially available, and for example, the Kaneka Cell Concentration and Washing System (manufactured by Kaneka Corporation) can be used.

The washing step can be performed at about 4°C to room temperature. For example, when lymphocytes expansion cultured in a 3 L bioreactor are washed using an automatic concentration and washing device, it is expected that it will take about 2 hr from cell recovery after culture to completion of the washing step. However, according to the washing method of the present invention, even if lymphocytes are suspended in the washing solution of the present invention and left standing at room temperature for 4 hr, the decrease in the viable cell recovery rate and cell survival rate after freezing and thawing and recovery culture is remarkably suppressed compared to when the lymphocytes are suspended in a physiological aqueous solution that does not contain bicarbonate ion, such as saline. Therefore, the washing solution and washing method of the present invention are extremely useful for use in the washing step (step of removing medium components) of lymphocytes after expansion culture.

### [II] The cryopreservation method of the present invention and the cryopreservation solution of the present invention

When lymphocytes obtained by the washing method of the present invention are suspended in a cryopreservation solution containing a physiological aqueous solution and cryopreserved, the viable cell recovery rate after thawing is remarkably improved compared to when the lymphocytes are frozen and stored in a commercially available cryopreservation solution that is widely used, such as STEM-CELLBANKER (registered trademark) and CryoStor CS10. Therefore, the present invention also provides a cryopreservation method of lymphocytes (hereinafter sometimes referred to as the "cryopreservation method of the present invention"), which includes suspending and freezing lymphocytes treated by the above-mentioned washing method of the present invention in a cryopreservation solution containing a physiological aqueous solution.

It has been known that washing cultured cells with bicarbonate Ringer's solution (containing sugars such as glucose and/or HuSA) suppresses the decrease in cell recovery rate and survival rate due to the washing step. However, the present inventors have surprisingly found for the first time that washing lymphocytes with a bicarbonate ion-containing physiological aqueous solution (preferably containing neither sugars nor HuSA) improves the cell recovery rate and survival rate after thawing and recovery culture, compared to washing with a physiological aqueous solution not containing bicarbonate ion, even when the lymphocytes are subsequently cryopreserved under the same conditions. That is, the lymphocytes obtained by the washing method of the present invention have novel and particularly remarkable unique characteristics compared to lymphocytes obtained by conventional washing methods; however, it is impossible or almost impractical to distinguish the two in terms of structure or properties.

The physiological aqueous solution used in the cryopreservation method of the present invention is not particularly limited as long as it improves the recovery rate of viable cells after thawing when lymphocytes obtained by the washing method of the present invention are suspended in a cryopreservation solution containing the physiological aqueous solution and cryopreserved. Any physiological aqueous solution such as those exemplified in the washing method of the present invention can be mentioned, and saline or a bicarbonate ion-containing physiological aqueous solution is preferred and saline or bicarbonate Ringer's solution is more preferred.

The concentration of the physiological aqueous solution in the cryopreservation solution used in the cryopreservation method of the present invention is, for example, in the case of saline, for example, 25 to 50% (v/v), preferably 30 to 45% (v/v), more preferably 32 to 41% (v/v), but is not limited thereto. Furthermore, when the physiological aqueous solution is bicarbonate Ringer's solution, its concentration in the cryopreservation solution is, for example, 35 to 75% (v/v), preferably 40 to 70% (v/v), more preferably 43 to 67% (v/v), but is not limited thereto.

The cryopreservation solution used in the cryopreservation method of the present invention preferably contains one or more cryoprotectants. Examples of cryoprotectants include, but are not limited to, dextran, DMSO, hydroxyethyl starch (HES), ethylene glycol, glycerol, trehalose, and the like. Dextran and DMSO are preferred.

Dextran is a polysaccharide (C₆H₁₀O₅)ₙ consisting of D-glucose and having an α1→6 bond as the main chain. The molecular weight of dextran is not particularly limited as long as it does not penetrate into cells, and examples of the molecular weight of dextran include a weight average molecular weight (Mw) of 40,000 (dextran 40) to 70,000 (dextran 70). Dextran can be produced by known methods such as chemical synthesis, production by microorganisms, and production by enzymes, and commercially available products may also be used. Examples of commercially available products of dextran include Dextran 40 (manufactured by Meito Sangyo Co., Ltd.) and Dextran 70 (manufactured by Tokyo Chemical Industry Co., Ltd.).

Alternatively, derivatives such as dextran sulfate, carboxylated dextran, and diethylaminoethyl (DEAE)-dextran can also be used.

Dextran or a derivative thereof may be in a free form or in the form of a salt. Examples of the salts thereof include acid addition salts with inorganic acids such as hydrochloride, hydrobromide, hydroiodide, phosphate, nitrate, sulfate, and acetate; acid addition salts with organic acids such as propionate, toluenesulfonate, succinate, oxalate, lactate, tartrate, glycolate, methanesulfonate, butyrate, valerate, citrate, fumarate, maleate, and malate; metal salts such as sodium salt, potassium salt, and calcium salt; and basic salts such as ammonium salt and alkylammonium salt. These salts may form hydrates or solvates, and may be used alone or in appropriate combination of two or more.

The concentration of dextran contained in the cryopreservation solution is, for example, 3 to 12% (w/v), preferably 3 to 11% (w/v), more preferably 3.5 to 10.5% (w/v), but is not limited thereto. The preferred concentration range may vary depending on the type of physiological aqueous solution to be used.

In order to avoid adverse effects on cells, it is preferable to use DMSO of as high purity as possible. The concentration of DMSO contained in the cryopreservation solution is, for example, 3-15% (w/v), preferably 5-12% (w/v), more preferably 7.5-12% (w/v), but is not limited thereto. The preferred concentration range may vary depending on the type of physiological aqueous solution to be used.

The cryopreservation solution to be used in the cryopreservation method of the present invention may contain one or more sugars. Examples of sugars include, but are not limited to, hexoses such as allose, altrose, glucose, mannose, gulose, idose, galactose, talose, precose, fructose, sorbose, tagalose, fucose, fuculose, and rhamnose; pentoses such as ribulose, xylulose, ribose, arabinose, xylose, and lyxose; disaccharides such as sucrose and maltose; oligosaccharides such as raffinose and stachyose; and polysaccharides such as starch and glycogen. Glucose is preferred.

The concentration of sugars to be contained in the cryopreservation solution is, for example, but not limited to, 0.03 to 1.2% (w/v), preferably 0.04 to 0.8% (w/v), more preferably 0.05 to 0.7% (w/v). The preferred concentration range may vary depending on the type of physiological aqueous solution to be used.

The cryopreservation solution to be used in the cryopreservation method of the present invention may contain one or more proteins. Examples of proteins include, but are not limited to, plasma-derived proteins such as serum albumin (e.g., HuSA, bovine serum albumin (BSA)) and serum globulin. HuSA is preferred. Plasma-derived proteins may be purified from animal plasma or recombinantly produced.

The concentration of the protein contained in the cryopreservation solution is, but is not limited to, 0.5 to 5% (w/v), preferably 1 to 4% (w/v), more preferably 1.2 to 3.5% (w/v). The preferred concentration range may vary depending on the type of physiological aqueous solution to be used

In one preferred embodiment, the cryopreservation solution to be used in the cryopreservation method of the present invention contains a physiological aqueous solution selected from saline or bicarbonate Ringer's solution, dextran, DMSO, glucose, and HuSA.

When the physiological aqueous solution is saline, the composition of the cryopreservation solution may be
saline 30 - 45% (v/v)
dextran 7 - 11% (w/v)
glucose 0.04 - 0.8% (w/v)
DMSO 5 - 12% (v/v)
HuSA 1 - 4% (w/v);
preferably,
saline 32 - 41% (v/v)
dextran 8 - 10% (w/v)
glucose 0.04 - 0.7% (w/v)
DMSO 5 - 11% (v/v)
HuSA 1.5 - 3% (w/v).

On the other hand, when the physiological aqueous solution is a bicarbonate Ringer's solution, the composition of the cryopreservation solution may be
bicarbonate Ringer's solution 40 to 70% (v/v)
dextran 3 - 11% (w/v)
glucose 0.04 - 0.8% (w/v)
DMSO 5 - 12% (v/v)
HuSA 1 - 4% (w/v);
preferably,
bicarbonate Ringer's solution 43 - 67% (v/v)
dextran 3.5 - 10.5% (w/v)
glucose 0.05 - 0.7% (w/v)
DMSO 7.5 - 12% (v/v)
HuSA 1.2 - 3.54% (w/v).

As mentioned above, there are reports of the use of a solution containing some of DMSO, dextran, glucose, HuSA, bicarbonate, and a physiological aqueous solution (e.g., saline, Ringer's solution) as a cryopreservation solution for lymphocytes, but a cryopreservation solution containing all of the aforementioned components or consisting of the above components has not yet been disclosed.

Therefore, the present invention also provides a cryopreservation solution for lymphocytes (hereinafter sometimes referred to as the "cryopreservation solution of the present invention") that contains a bicarbonate ion-containing physiological aqueous solution, dextran, glucose, DMSO, and HuSA.

The bicarbonate ion-containing physiological aqueous solution, dextran, glucose, DMSO, and HuSA, which are components of the cryopreservation solution of the present invention, can be those described for the cryopreservation method of the present invention. The bicarbonate ion-containing physiological aqueous solution is preferably bicarbonate Ringer's solution.

When the bicarbonate ion-containing physiological aqueous solution is bicarbonate Ringer's solution, the composition of the cryopreservation solution of the present invention may be generally as follows.
bicarbonate Ringer's solution 40 - 70% (v/v)
dextran 3 - 11% (w/v)
glucose 0.04 - 0.8% (w/v)
DMSO 5 - 12% (v/v)
HuSA 1 - 4% (w/v);
more preferably,
bicarbonate Ringer's solution 43 - 67% (v/v)
dextran 3.5 - 10.5% (w/v)
glucose 0.05 - 0.7% (w/v)
DMSO 7.5 - 12% (v/v)
HuSA 1.2 - 3.5% (w/v)

In the cryopreservation method of the present invention, first, the washing solution is removed from the cell concentrate of lymphocytes obtained by performing the above-mentioned washing method of the present invention, and the lymphocytes are resuspended in any of the above-mentioned cryopreservation solutions. For example, the cell concentrate is collected in a centrifuge tube or the like, the cells are pelleted by centrifugation, and then the cryopreservation solution can be added to suspend the cells. Alternatively, the washing solution can be continuously replaced with the cryopreservation solution by simultaneously feeding the cryopreservation solution and removing the washing solution.

The obtained cell suspension can be placed in a cryopreservation tube, frozen in a freezer at -80°C, and then stored in a nitrogen tank in the gas or liquid phase, but the process is not limited to this. For example, the cell suspension is dispensed into cryopreservation vials by using an automatic dispensing device, and the freezing step can be optimized by performing a programmable freezing using, for example, a freezer that can control the cooling rate.

For example, it is estimated that it takes about 2 hours to replace the washing solution of the cell concentrate, obtained by subjecting lymphocytes that have been expansion cultured in a 3 L bioreactor to the washing method of the present invention, with a cryopreservation solution, dispense the obtained cell suspension into a cryopreservation container, and start the freezing treatment. This operation is performed at about 4°C to room temperature. Therefore, the cryopreservation solution is required not only to protect the cells from damage during cryopreservation and thawing, but also to stably preserve the cells at about 4°C to room temperature from the time when the cells are resuspended in the cryopreservation solution after washing until the freezing treatment is started. As shown in the below-mentioned examples, when lymphocytes after washing are suspended in a cryopreservation solution and left standing at room temperature for 4 hr, the decrease in the viable cell recovery rate over time is remarkably suppressed when cryopreservation is performed using a cryopreservation solution containing bicarbonate Ringer's solution, compared to SCB, an existing cryopreservation solution. Therefore, the cryopreservation method and cryopreservation solution of the present invention are extremely useful for cryopreservation of lymphocytes.

### [III] The lymphocyte-containing composition of the present invention, the diluent for transplantation of the present invention, a kit containing them, a method for preparing lymphocytes for transplantation, which uses same, and a lymphocyte preparation for transplantation, which is obtained thereby

The frozen lymphocyte-containing composition obtained by the above-mentioned cryopreservation method of the present invention is remarkably improved in the recovery rate of viable cells and cell survival rate after thawing and recovery culture, compared to the case where an existing cryopreservation solution is used. That is, the lymphocyte-containing composition affords an advantageous effect as lymphocytes for transplantation. Therefore, the present invention also provides a lymphocyte-containing composition (hereinafter sometimes referred to as "the lymphocyte-containing composition of the present invention") that contains lymphocytes treated with a bicarbonate ion-containing physiological aqueous solution and the above-mentioned cryopreservation solution of the present invention.

As shown in the examples below, a frozen lymphocyte-containing composition obtained by the washing method and cryopreservation method of the present invention was thawed, diluted with a diluent for transplantation containing a bicarbonate ion-containing physiological aqueous solution, and left standing at room temperature for 4 hr, after which recovery culture was performed. Then, the cell survival rate after culture was evaluated, and a higher cell survival rate was obtained compared to dilution with a physiological aqueous solution not containing bicarbonate ion (e.g., saline, acetated Ringer). In other words, a bicarbonate ion-containing physiological aqueous solution is extremely useful as a medium for transplantation. Therefore, the present invention also provides a diluent for transplantation, which is a diluent for transplantation for the lymphocyte-containing composition of the present invention and contains a bicarbonate ion-containing physiological aqueous solution. As the bicarbonate ion-containing physiological aqueous solution, a solution used in the aforementioned washing solution of the present invention can be appropriately selected and used, but preferably, it is bicarbonate Ringer's solution. Furthermore, the present invention provides a kit for preparing lymphocytes for transplantation, which contains the lymphocyte-containing composition of the present invention and a diluent for transplantation containing a bicarbonate ion-containing physiological aqueous solution.

In the aforementioned kit for preparing lymphocytes for transplantation, the lymphocyte-containing composition may be provided in a frozen state. For example, the lymphocyte-containing composition may be transported from the CPC to the hospital in a frozen state, and after thawing, the composition may be diluted and suspended in a diluent for transplantation containing a bicarbonate ion-containing physiological aqueous solution to prepare a lymphocyte preparation for transplantation, which can be stably maintained at room temperature until transplantation. Alternatively, in another embodiment, immediately after thawing in the CPC, the frozen lymphocyte-containing composition may be diluted and suspended in a diluent for transplantation containing a bicarbonate ion-containing physiological aqueous solution, and the lymphocytes may be transported to the hospital at room temperature in an unfrozen state, and transplantation may be performed immediately after arrival.

Therefore, the present invention also provides a method for preparing lymphocytes for transplantation, which includes diluting the lymphocyte-containing composition of the present invention, which has been frozen and then thawed, with a diluent for transplantation containing a bicarbonate ion-containing physiological aqueous solution. Furthermore, the present invention provides a lymphocyte preparation for transplantation which is obtained by the method.

The thus-obtained lymphocyte preparation for transplantation can be administered to a patient, for example, a cancer patient, by a method known per se, such as the form of administration, method of use, and dosage used in adoptive immunotherapy and donor lymphocyte infusion therapy.

The present invention is explained in more detail in the following by referring to examples, but these are merely illustrative and the present invention is not limited thereto.

### [Example]

### [Example 1] Production of iPS cell-derived NK cell

Human umbilical cord blood-derived iPS cell stock was thawed, seeded in a flask, and cultured in 37°C, 5% CO₂ environment, and the cells were collected and seeded in a three-dimensional culture device. After seeding, perfusion culture was performed through a membrane filtration filter to form 3D spheres of a certain size. Thereafter, a differentiation induction medium containing additive factors such as BMP4 was added to the cells, and HPC was induced by continuing 3D culture by perfusion culture for 12-17 days. Furthermore, the obtained HPC was seeded in a three-dimensional culture device using NK differentiation induction medium (AIM V Serum Free Medium (Thermo Fisher Scientific) added with 5% FBS, and IL-15, IL-7, SCF, and Flt3L at a final concentration of 50 ng/mL) and perfusion cultured for about 40 days to produce a large amount of NK cells having high physiological activity.

### [Example 2] Comparison of washing solutions in lymphocyte washing step

### (1) Preparation of NK cell

The NK cells obtained in Example 1 were centrifuged at 300xg for 5 min, the culture medium supernatant was removed, and the cells were collected and suspended in five types of physiological aqueous solutions [(1) saline (manufactured by Otsuka Pharmaceutical Factory, Inc.), (2) Veen-F (acetated Ringer's solution; manufactured by Fuso Pharmaceutical Industries, Ltd.), (3) Hespander (hydroxyethyl starch and glucose added lactated Ringer's solution; manufactured by Otsuka Pharmaceutical Factory, Inc.), (4) HBSS(+) (balanced salt solution), (5) HBSS(-) (balanced salt solution)] to a concentration of 2.2 x 10⁶ cells/mL (the electrolyte composition of each physiological aqueous solution is shown in Table 1). The NK cell suspension was placed in a 15 mL centrifuge tube and left standing at room temperature. The standing treatment was used to simulate the process time of the concentration and washing step, with 0 hr set as comparative example and 2 and 4 hr set as examples.

**[Table 1]**

| electrolyte concentration mmol/L | OTSUKA NORMAL SALINE (0.9% saline) | Veen-F (acetated Ringer's solution) | Hespander (hydroxyethyl starch and glucose added lactated Ringer's solution) | HBSS (+) (balanced salt solution) | HBSS (-) (balanced salt solution) |
|---|---|---|---|---|---|
| Na | 154 | 130 | 105.6 | 142.77 | 142.77 |
| K | - | 4 | 4 | 5.77 | 5.77 |
| Ca | - | 1.5 | 1.35 | 1.26 | - |
| Mg | - | - | - | 0.90 | - |
| Cl | 154 | 109 | 92.3 | 146.77 | 143.26 |
| lactic acid | - | - | 20 | - | - |
| acetic acid | - | 28 | - | - | - |
| hydrogen carbonate | - | - | - | 4.17 | 4.17 |
| phosphoric acid | - | - | - | 0.78 | 0.78 |

As shown in Table 1, the bicarbonate ion in the balanced salt solution HBSS composition is not contained in "saline", "acetated Ringer's solution", or "lactate Ringer's solution with added hydroxyethyl starch and glucose".

### (2) Cryopreservation of NK cell samples after standing treatment

After standing for 0 hr (immediately after suspending), 2 hr, and 4 hr, the NK cell suspension was centrifuged at 300xg for 5 min, the supernatant was removed, and the cells were resuspended in STEM-CELLBANKER (registered trademark) GMP grade (SCB, manufactured by ZENOGEN PHARMA CO., LTD.) to a concentration of 5.0 x 10⁶ cells/mL. The cell suspension was then dispensed into freezing containers, transferred to freezing storage container BICELL (manufactured by NIHON FREEZER CO., LTD.), and cryopreserved at -80°C.

### (3) Measurement of cell recovery rate and cell survival rate after thawing and recovery culture

NK cells were thawed and reseeded in a 6-well plate at 1.0 x 10⁶ cells/mL using the medium used for NK cell production (AIM V Serum Free Medium (Thermo Fisher Scientific) with 5% FBS, and IL-15, IL-7, SCF, and Flt3L added to a final concentration of 50 ng/mL). Recovery culture was performed for 4 days in an incubator at 37°C, 5% CO₂. The viable cell density in the well was then measured, and the viable cell recovery rate (see formula (I)) and cell survival rate were calculated. Viable cell recovery rate = (total number of viable cells after thawing and after completion of culture/total number of cryopreserved viable cells) × 100

The results are shown in Table 2.

**[Table 2]**

| type of washing solution | standing time | immediately after thawing Day0 | | completion of culture Day4 | |
|---|---|---|---|---|---|
| | | viable cell recovery rate (%) | cell survival rate (%) | viable cell recovery rate (%) | cell survival rate (%) |
| saline | 0hr | 65.7% | 96.9% | 59.6% | 96.0% |
| | 2hr | 61.0% | 92.3% | 29.6% | 88.8% |
| | 4hr | 47.4% | 83.2% | 25.4% | 88.9% |
| Veen-F (acetated Ringer's solution) | 0hr | 60.8% | 97.1% | 41.8% | 94.1% |
| | 2hr | 65.0% | 96.7% | 38.2% | 93.5% |
| | 4hr | 63.8% | 96.6% | 45.4% | 96.8% |
| Hespander (hydroxyethyl starch and glucose added lactated Ringer's solution) | 0hr | 43.2% | 95.3% | 39.2% | 96.2% |
| | 2hr | 39.2% | 92.4% | 16.8% | 93.0% |
| | 4hr | 46.5% | 94.4% | 24.4% | 95.9% |
| HBSS (+) (balanced salt solution) | 0hr | 67.8% | 97.4% | 49.4% | 96.1% |
| | 2hr | 66.6% | 97.6% | 47.3% | 95.3% |
| | 4hr | 63.0% | 95.3% | 53.6% | 95.3% |
| HBSS (-) (balanced salt solution) | 0hr | 61.9% | 96.4% | 55.7% | 95.8% |
| | 2hr | 60.4% | 95.3% | 50.9% | 96.4% |
| | 4hr | 54.0% | 92.9% | 36.7% | 94.1% |

The results in Table 2 show that for NK cells suspended in saline, the viable cell recovery rate and cell survival rate of NK cells immediately after thawing and after completion of culture in a 4-hr standing treatment are remarkably lower than those in 0 hr (no standing treatment) and 2-hr standing treatments. In practice, the process time for the cell recovery and washing steps is set to within about 2 hr, but the process time up to the cryopreservation step by further adding a cryopreservation solution is assumed to be about 4 hr. By comparison of the viable cell recovery rate and cell survival rate after completion of recovery culture of NK cells subjected to the standing treatment for 4 hr, it was confirmed that NK cells suspended in a balanced salt solution such as HBSS had a higher viable cell recovery rate and a higher cell survival rate than those of NK cells suspended in saline.

From the above-mentioned results, it was considered that a solution containing bicarbonate ion is suitable for recovering NK cells and maintaining the survival rate thereof in the buffer replacement or washing step of the suspension containing recovered NK cells.

### [Example 3] Comparison of washing solutions in lymphocyte concentration and washing step

### (1) Preparation and standing treatment of NK cell suspension

The NK cells obtained in Example 1 were centrifuged at 300xg for 5 min, the culture medium supernatant was removed, and the cells were collected and suspended in three types of physiological aqueous solutions [(1) saline, (2) Lactec (lactated Ringer's solution; manufactured by Otsuka Pharmaceutical Factory, Inc.), (3) BICANATE (bicarbonate Ringer's solution; manufactured by Otsuka Pharmaceutical Factory, Inc.)] to a concentration of 2.2x10⁶ cells/mL (the electrolyte composition of each physiological aqueous solution is shown in Table 3). The NK cell suspension was placed in a 15 mL centrifuge tube and left standing at room temperature. The standing treatment was used to simulate the process time of the concentration and washing step, with 0 hr set as comparative example and 2 and 4 hr set as examples.

As shown in Table 3, Bicanate, which is a bicarbonate Ringer's solution, contains bicarbonate ion.

**[Table 3]**

| electrolyte concentration mmol/L | OTSUKA NORMAL SALINE (0.9% saline) | Lactec (lactated Ringer's solution) | BICANATE (bicarbonate Ringer's solution) |
|---|---|---|---|
| Na | 154 | 130 | 130 |
| K | - | 4 | 4 |
| Ca | - | 1.5 | 1.5 |
| Mg | - | - | 1 |
| Cl | 154 | 109 | 109 |
| lactic acid | - | 28 | - |
| acetic acid | - | - | - |
| hydrogen carbonate | - | - | 28 |
| phosphoric acid | - | - | - |

### (2) Cryopreservation of NK cell samples after standing treatment

After standing for 0 hr (immediately after suspending), 2 hr, and 4 hr, the NK cell suspension was centrifuged at 300xg for 5 min, the supernatant was removed, and the cells were resuspended in STEM-CELLBANKER (registered trademark) GMP grade (SCB) to a concentration of 5.0 x 10⁶ cells/mL. The cell suspension was then dispensed into freezing containers, transferred to freezing storage container BICELL, and cryopreserved at -80°C.

### (3) Measurement of viable cell recovery rate and cell survival rate after thawing and recovery culture

NK cells were thawed and reseeded in a 6-well plate at 1.0 x 10⁶ cells/mL using the medium used for NK cell production in Example 1 (AIM V Serum Free Medium (Thermo Fisher Scientific) with 5% FBS, and IL-15, IL-7, SCF, and Flt3L added to a final concentration of 50 ng/mL). Recovery culture was performed for 3 days in an incubator at 37°C, 5% CO₂. The viable cell density in the well was then measured, and the viable cell recovery rate (see formula (I)) and cell survival rate were calculated.
The results are shown in Table 4.

**[Table 4]**

| type of washing solution | standing time | immediately after thawing Day0 | | completion of culture Day3 | |
|---|---|---|---|---|---|
| | | viable cell recovery rate (%) | cell survival rate (%) | viable cell recovery rate (%) | cell survival rate (%) |
| saline | 0hr | 79.0% | 97.5% | 53.0% | 97.8% |
| | 2hr | 77.8% | 97.1% | 61.4% | 99.3% |
| | 4hr | 57.1% | 84.4% | 30.4% | 96.9% |
| Lactec (lactated Ringer's solution) | 0hr | 74.2% | 98.4% | 66.3% | 98.8% |
| | 2hr | 74.9% | 97.8% | 45.8% | 97.9% |
| | 4hr | 66.3% | 91.7% | 32.1% | 96.6% |
| BICANATE (bicarbonate Ringer's solution) | 0hr | 83.4% | 98.9% | 52.5% | 98.8% |
| | 2hr | 77.4% | 98.8% | 71.7% | 98.8% |
| | 4hr | 75.4% | 99.2% | 68.5% | 99.1% |

The results in Table 4 show that for NK cells suspended in saline, the viable cell recovery rate and cell survival rate of NK cells immediately after thawing and after completion of culture in a 4-hr standing treatment are remarkably lower than those in 0 hr (no standing treatment) and 2-hr standing treatments.

By comparison of the viable cell recovery rate and cell survival rate after completion of recovery culture in the case of standing for 4 hr, it was confirmed that NK cells suspended in BICANATE, which is a bicarbonate Ringer's solution, had a higher viable cell recovery rate and a higher cell survival rate than those of NK cells suspended in saline.

From the above-mentioned results, it was found that the viable cell recovery rate and survival rate of NK cells are optimized by buffer replacement or washing of the NK cell suspension with bicarbonate Ringer's solution, which has a buffering effect and contains bicarbonate ion, compared to saline, which has no buffering effect, and lactate Ringer's solution, which does not contain bicarbonate ion.

### [Example 4] Cryopreservation solution for lymphocytes (1) Mixing and freezing of NK cell suspension and cryopreservation solution

The culture solution containing NK cells obtained in Example 1 was centrifuged at 300xg for 5 min, and the medium supernatant was removed. The NK cell precipitate was loosened by tapping and suspended in saline or bicarbonate Ringer's solution, and then cryopreservation solutions of respective compositions shown in Table 5 or Table 6 were added and mixed well. After adding the cryopreservation solutions, each cell suspension was dispensed into freezing containers, transferred to cryopreservation containers such as Mr. Frosty (manufactured by Nalgene) or BICELL, and frozen at -80°C. To compare the performance of the cryopreservation solutions, cells suspended in STEM-CELLBANKER (registered trademark) GMP grade (SCB) were prepared and cryopreserved as a control sample.

**[Table 5]**

| Composition of saline-containing cryopreservation solution and viable cell recovery rate (SCB relative value) | | | | | | | | |
|---|---|---|---|---|---|---|---|---|
| cryopreservation solution | SCB | CS10 | saline | Dex40 | Glucose | DMSO | HuSA | viable cell recovery rate (SCB relative value) |
| SCB | 100 | - | - | - | - | - | - | 1.00 |
| CS10 | - | 100 | - | - | - | - | - | 0.80 |
| H-16 | - | - | 36.8 | 8.06 | 0.090 | | 10.0 2.75 | 1.16 |
| H-33 | - | - | 32.7 | 9.68 | 0.360 | 9.1 | 1.75 | 1.15 |
| H-35 | - | - | 37.0 | 8.72 | 0.675 | 9.1 | 1.75 | 1.24 |
| H-38 | - | - | 40.8 | 8.72 | 0.360 | 6.0 | 1.75 | 1.21 |
| H-39 | - | - | 36.5 | 9.68 | 0.045 | 6.0 | 1.75 | 1.23 |
| H-64 | - | - | 37.7 | 8.72 | 0.360 | 9.1 | 1.75 | 1.25 |

| | | | | | | | | |
|---|---|---|---|---|---|---|---|---|
| SCB relative value: (viable cell recovery rate of each sample/viable cell recovery rate of SCB)x100 | | | | | | | | |

**[Table 6]**

| Composition of bicarbonate Ringer's solution-containing cryopreservation solution and viable cell recovery rate (SCB relative value) | | | | | | | | |
|---|---|---|---|---|---|---|---|---|
| cryopreservation solution | SCB | saline | bicarbonate Ringer's solution | Dex40 | Glucose | DMSO | HuSA | viable cell recovery rate (SCB relative value) |
| SCB | 100 | - | - | - | - | - | - | 1.00 |
| H-16 | - | 36.8 | - | 8.06 | 0.09 | 10 | 2.75 | 1.08 |
| H-49 | - | - | 53.3 | 6.72 | 0.1 | 8.5 | 2.5 | 1.16 |
| H-51 | - | - | 66.1 | 3.60 | 0.2 | 11 | 1.875 | 1.24 |
| H-53 | - | - | 54 | 5.76 | 0 | 12 | 2.5 | 1.05 |
| H-55 | - | - | 68 | 4.32 | 0.25 | 7.5 | 1.5 | 1.03 |
| H-59 | - | - | 44 | 7.44 | 0.25 | 11 | 3.375 | 1.11 |
| H-88 | - | - | 60.2 | 4.30 | 0.09 | 9.9 | 2.95 | 1.15 |

| | | | | | | | | |
|---|---|---|---|---|---|---|---|---|
| SCB relative value: (viable cell recovery rate of each sample/viable cell recovery rate of SCB)x100 | | | | | | | | |

### (2) Thawing and recovery culture of NK cells after cryopreservation

The frozen NK cells obtained in the above-mentioned step (1) were thawed and suspended in the medium used for NK cell production in Example 1 (AIM V Serum Free Medium (Thermo Fisher Scientific) with 5% FBS, and IL-15, IL-7, SCF, and Flt3L added to a final concentration of 50 ng/mL), and reseeded in a 12-well plate at 1.0 x 10⁶ cells/mL. Then, recovery culture was performed for 3 days in an incubator at 37°C, 5% CO₂. Thereafter, the viable cell density and cell survival rate in the wells were measured, and the viable cell recovery rate in each cryopreservation solution (see formula (I)) was calculated as a relative value to the viable cell recovery rate of cells frozen using SCB (SCB relative value).

The results obtained using a cryopreservation solution containing saline are shown in Table 5 (SCB relative value on Day 3 after completion of culture), and the results obtained using a cryopreservation solution containing Ringer's bicarbonate solution are shown in Table 6 (SCB relative value on Day 3 after completion of culture).

### (3) Results

### (3-1) In the case of cryopreservation solution containing saline

When cryopreserving the NK cells prepared in Example 1 by using a cryopreservation solution containing saline, plural compositions could be obtained that had a remarkably high viable cell recovery rate after thawing than the existing cryopreservation solutions SCB and CryoStor CS10 (CS10; manufactured by Charles River Laboratories Cell Solutions, Inc.). The preferred compositions were saline; 32.7-40.8% (v/v), Dextran40 (Dex40; manufactured by Meito Sangyo Co., Ltd.); 8.06-9.68% (w/v), Glucose (manufactured by TERUMO CORPORATION); 0.045-0.675% (w/v), CryoSure DMSO (DMSO; manufactured by WAK-Chemie Medical GmbH); 6.0-10.0% (v/v), and human serum albumin (HuSA; manufactured by Japan Blood Products Organization); 1.75-2.75% (w/v). (Table 5)

### (3-2) In the case of cryopreservation solution containing bicarbonate Ringer's solution

When cryopreserving the NK cells prepared in Example 1 by using a cryopreservation solution containing bicarbonate Ringer's solution, plural compositions could be obtained that had a remarkably high viable cell recovery rate after thawing than the existing cryopreservation solutions SCB. The preferred compositions were bicarbonate Ringer's solution; 44.0-66.1% (v/v), Dextran40; 3.60-7.44% (w/v), Glucose; 0.09-0.25% (w/v), DMSO; 8.5-11.0% (v/v), HuSA; 1.875-3.375% (w/v). (Table 6)

### [Example 5] Study of cryopreservation solution for time stability of NK cell

### (1) Mixing and freezing of NK cell suspension and cryopreservation solution

The culture solution containing NK cells prepared in Example 1 was centrifuged at 300xg for 5 min, and the medium supernatant was removed. The NK cell precipitate was dissolved by tapping and suspended in saline or bicarbonate Ringer's solution, and then cryopreservation solutions of respective compositions shown in Table 7 were added and mixed well. The cell suspensions added with the cryopreservation solution were dispensed into freezing containers, left standing at room temperature for the desired time (1, 2, 4 hr), then transferred to a freezing container (Mr. Frosty or BICELL) and frozen at - 80°C.

### (2) Thawing and recovery culture of NK cells after cryopreservation

After thawing the NK cells, they were reseeded in a 12-well plate at 1.0 x 10⁶ cells/mL using the NK cell expansion culture medium of Example 1, and recovery culture was performed for 3 days in an incubator at 37°C, 5% CO₂. Thereafter, the viable cell density and cell survival rate in the wells were measured, and the viable cell recovery rate (see formula (I)) was calculated as the SCB relative value. The results are shown in Table 7 (SCB relative value on Day 3 after completion of culture).

**[Table 7]**

| Composition of cryopreservation solution and stability in standing at room temperature (SCB relative value) | | | | | | | | | | | |
|---|---|---|---|---|---|---|---|---|---|---|---|
| | composition of cryopreservation solution | | | | | | | viable cell recovery rate at each standing time (SCB relative value) | | | |
| cryopreservation solution | SCB | saline | bicarbonate Ringer's solution | Dex40 | Glucose | DMSO | HuSA | 0h | 1h | 2h | 4h |
| SCB | 100 | - | - | - | - | - | - | 1.00 | 0.70 | 0.45 | 0.35 |
| H-95 | - | 25.22 | 20 | 8.06 | 0.09 | 10 | 2.75 | 1.03 | 0.79 | 0.57 | 0.39 |
| H-88 | - | - | 60.20 | 4.30 | 0.09 | 9.90 | 2.95 | 1.11 | 0.94 | 0.73 | 0.51 |
| H-90 | - | - | 46.59 | 9.68 | 0.05 | 6.00 | 1.75 | 0.96 | 0.77 | 0.60 | 0.37 |
| H-51 | - | - | 66.10 | 3.60 | 0.20 | 11.00 | 1.88 | 1.08 | 0.89 | 0.64 | 0.51 |
| H-98 | - | - | 52.71 | 6.18 | 0.09 | 9.95 | 2.85 | 1.13 | 0.81 | 0.57 | 0.46 |
| H-99 | - | - | 51.34 | 6.93 | 0.32 | 10.75 | 2.10 | 1.06 | 0.79 | 0.56 | 0.40 |
| H-100 | - | - | 43.22 | 10.06 | 0.41 | 8.05 | 1.50 | 1.13 | 0.82 | 0.58 | 0.40 |
| H-101 | - | - | 44.59 | 9.31 | 0.18 | 7.25 | 2.25 | 1.02 | 0.90 | 0.64 | 0.37 |

| | | | | | | | | | | | |
|---|---|---|---|---|---|---|---|---|---|---|---|
| SCB relative value: (viable cell recovery rate of each sample/viable cell recovery rate of SCB)x100 | | | | | | | | | | | |

When the cells were left standing at room temperature for 0 hr to 4 hr after mixing with the cryopreservation solution, the viable cell recovery rate decreased over time. In contrast, when cryopreservation was performed using a cryopreservation solution containing bicarbonate Ringer's solution, a remarkably higher viable cell recovery rate could be maintained than when using the existing cryopreservation solution, SCB. In particular, the preferred compositions were bicarbonate Ringer's solution; 43.2-66.10% (v/v), Dextran40; 3.60-10.06% (w/v), Glucose; 0.05-0.41% (w/v), DMSO; 6.0-11.0% (v/v), HuSA; 1.50-2.95% (w/v). (Table 7)

### [Example 6] Diluent of NK cells after freezing and thawing

After thawing the NK cells, 0.1 mL of the cell suspension was diluted and suspended in a 10-fold amount of each physiological aqueous solution, and the stability was evaluated after standing at room temperature for 1, 2, and 4 hr. The medium for recovery culture was used as a control for the physiological aqueous solution. After standing, the cell suspension was centrifuged at 300xg for 5 min to remove the supernatant, then resuspended in 2 mL of the medium for NK cell expansion culture in Example 1, 1.9 mL was seeded on a 12-well plate, and recovery culture was performed in an incubator under the conditions of 37°C and 5% CO₂ for 3 days. Thereafter, the viable cell density in the well was measured, and the number of viable cells was counted. The physiological aqueous solutions used in the study were saline (manufactured by Otsuka Pharmaceutical Factory, Inc.), Veen-D (acetated Ringer's solution; manufactured by Fuso Pharmaceutical Industries, Ltd.), Veen-F (acetated Ringer's solution; manufactured by Fuso Pharmaceutical Industries, Ltd.), BICANATE (bicarbonate Ringer's solution; manufactured by Otsuka Pharmaceutical Factory, Inc.), and BICARBON (bicarbonate Ringer's solution; manufactured by AY PHARMACEUTICALS CO., LTD.) (the composition of each physiological aqueous solution is shown in Table 8).

**[Table 8]**

| composition | saline | Veen-F | Veen-D | BICANATE | BICARBON |
|---|---|---|---|---|---|
| glucose | | - | 5 | - | - |
| sodium chloride | 0.9 | 0.6 | 0.6 | 0.584 | 0.614 |
| potassium chloride | | 0.03 | 0.03 | 0.03 | 0.03 |
| calcium chloride hydrate | - | 0.02 | 0.02 | 0.022 | 0.022 |
| sodium acetate hydrate | | 0.38 | 0.38 | - | - |
| magnesium chloride | | suitable amount | suitable amount | 0.02 | 0.0102 |
| sodium bicarbonate | - | - | - | 0.235 | 0.21 |
| sodium citrate | - | - | - | 0.02 | 0.049 |
| hydrochloric acid | - | suitable amount | suitable amount | - | - |
| carbon dioxide | - | - | - | - | suitable amount |

The results of the experiment using saline, Veen-F, and Veen-D are shown in Table 9, the results of the experiment using saline and Bicanate in Table 10, and the results of the experiment using saline, BICANATE, and BICARBON are shown in Table 11.

**[Table 9]**

| physiological aqueous solution | standing time (hour) | number of viable cells (×10⁵ cells/mL) |
|---|---|---|
| medium | 0 | 8. 06 |
| saline | 0 | 6.67 |
| | 1 | 5.31 |
| | 2 | 4.8 |
| | 4 | 3.7 |
| Veen-F | 0 | 7. 18 |
| | 1 | 6.3 |
| | 2 | 5.26 |
| | 4 | 3. 99 |
| Veen-D | 0 | 4.74 |
| | 1 | 2.52 |
| | 2 | 1.49 |
| | 4 | 0.87 |

**[Table 10]**

| physiological aqueous solution | standing time (hour) | number of viable cells (×10⁵ cells/mL) |
|---|---|---|
| medium | 0 | 9.69 |
| saline | 0 | 8.85 |
| | 1 | 5. 29 |
| | 2 | 6.2 |
| | 4 | 4.25 |
| BICANATE | 0 | 9.79 |
| | 1 | 8.82 |
| | 2 | 8.04 |
| | 4 | 7.12 |

**[Table 11]**

| physiological aqueous solution | standing time (hour) | number of viable cells (×10⁵ cells/mL) |
|---|---|---|
| medium | 0 | 7.07 |
| saline | 0 | 6.71 |
| | 1 | 5.59 |
| | 2 | 4.5 |
| | 4 | 4.39 |
| BICANATE | 0 | 7.28 |
| | 1 | 6.82 |
| | 2 | 7.19 |
| | 4 | 5.18 |
| BICARBON | 0 | 7.04 |
| | 1 | 7.33 |
| | 2 | 7.06 |
| | 4 | 5.71 |

As shown in Tables 9-11, after thawing the NK cells cryopreserved by the method of Example 4, they were suspended in a physiological aqueous solution at a dilution of 10 times and left standing, after which the decrease in the number of viable cells recovered over time was evaluated. When bicarbonate Ringer's solution, such as BICANATE or BICARBON, was used, a remarkably higher number of viable cells was recovered than when saline, Veen-D, or Veen-F was used. It was clarified that bicarbonate Ringer's solution has a high viable cell number maintaining effect as a diluent for administering NK cell frozen preparations to patients.

### [Industrial Applicability]

According to the washing method and the cryopreservation method of the present invention, high cell recovery rates and survival rates can be maintained even after freezing and thawing, and lymphocytes (e.g., NK cell, T cell, etc.) for transplantation that maintain high physiological activity can be produced. Furthermore, the lymphocyte-containing composition and the diluent for transplantation of the present invention can maintain a high cell survival rate even when stored at room temperature after thawing until transplantation, and can provide a lymphocyte preparation more suitable for transplantation. Therefore, the present invention is extremely useful in cellular immunotherapy using lymphocytes, including cancer immunotherapy.

### (CROSS REFERENCES TO RELATED APPLICATION)

This application is based on a patent application No. 2022-185201 filed in Japan (filing date: November 18, 2022), the contents of which are incorporated in full herein by reference.

## Claims

1. A method for washing lymphocytes, comprising washing lymphocytes with a bicarbonate ion-containing physiological aqueous solution.

2. The method according to claim 1, wherein the lymphocyte is a T cell or NK cell.

3. The method according to claim 1, wherein the bicarbonate ion-containing physiological aqueous solution is bicarbonate Ringer's solution.

4. A method for cryopreserving lymphocytes, comprising suspending and freezing lymphocytes treated by the method according to claim 1 in a cryopreservation solution containing a physiological aqueous solution.

5. The method according to claim 4, wherein the physiological aqueous solution is a bicarbonate ion-containing physiological aqueous solution.

6. The method according to claim 5, wherein the bicarbonate ion-containing physiological aqueous solution is bicarbonate Ringer's solution.

7. A washing solution for lymphocytes, comprising a bicarbonate ion-containing physiological aqueous solution.

8. A cryopreservation solution for lymphocytes, comprising a bicarbonate ion-containing physiological aqueous solution, dextran, glucose, DMSO, and human serum albumin.

9. A lymphocyte-containing composition, comprising lymphocytes treated with a bicarbonate ion-containing physiological aqueous solution and the cryopreservation solution according to claim 8.

10. A kit for preparing lymphocytes for transplantation, comprising the lymphocyte-containing composition according to claim 9 and a diluent for transplantation containing a bicarbonate ion-containing physiological aqueous solution.

11. A diluent for transplantation for the lymphocyte-containing composition according to claim 9, the diluent comprising a bicarbonate ion-containing physiological aqueous solution.

12. A method for preparing lymphocytes for transplantation, comprising diluting the lymphocyte-containing composition according to claim 9, which has been cryopreserved and then thawed, with a diluent for transplantation containing a bicarbonate ion-containing physiological aqueous solution.

13. A lymphocyte preparation for transplantation, which is obtained by the method according to claim 12.
